# EUROPEAN PATENT APPLICATION

(11) **EP 1 944 297 A1**
(43) Date of publication of application: **16.07.2008**
(21) Application number: 07000304.1
(22) Date of filing: 09.01.2007
(51) Int. Cl.: C07D 231/14, A61K 31/455, A61P 35/00

(54) **Solid and crystalline rimonabant and processes for preparation, and pharmaceutical composition thereof**

(71) Applicant: Vértessy, Miklós, Mátyás királa út 23 H 1125 Budapest (HU)
(72) Inventor: Vértessy, Miklós, Mátyás királa út 23 H 1125 Budapest (HU)
(74) Representative: Winkler, Andreas Fritz Ernst

(57) **Abstract**

The present invention relates to the solid state chemistry of N-piperidino-5-(4-chlorophenyl)-1-(2,4-dichlorophenyl)-4-methyl-3-pyrazolecarboxamide which international nonproprietary name is rimonabant, its preparation and pharmaceutical compositions containing said novel polymorph.

Thus, the subject of the present invention is a novel polymorphic form of rimonabant, called form III, it also relates to methods for preparing rimonabant in its polymorphic form III, and an amorphous form

## Description

### FIELD OF THE INVENTION

The present invention relates to the solid state chemistry of N-piperidino-5-(4-chlorophenyl)-1-(2,4-dichlorophenyl)-4-methyl-3-pyrazolecarboxamide which international nonproprietary name is rimonabant, its preparation and pharmaceutical compositions containing said novel polymorph.

Thus, the subject of the present invention is a novel polymorphic form of rimonabant, called form III, it also relates to methods for preparing rimonabant in its polymorphic form III, and an amorphous form.

### BACKGROUND OF THE INVENTION

The chemical structure of Rimonabant is the following:

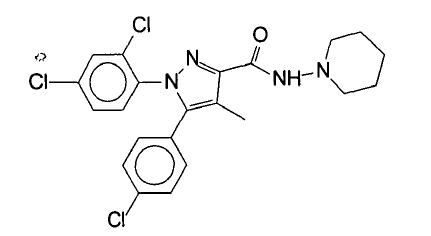

Rimonabant is an antagonist of the CB₁ cannabinoid receptors which was described for the first time in European patent: EP 0 656 354. The mentioned patent EP 0 656 354 makes no reference to the existence of specific polymorphic forms of rimonabant.

US patent N°2005/0043356 A1 discloses a new solid phase named form II, because the solid form obtained in the previous patent (EP 0656354) was named form I.

The processes to obtain each solid phase (I or II) were described in the aforementioned documents (US patent N°2005/0043356 A1 and EP 0656354). Form II is obtained by crystallization in methylcyclohexane in the pure state or containing 1 to 10 % of water by volume. This solvent is very flammable and its use is generally avoided in industrial scale. On the other hand, form I only could be obtained from the reaction mixture during the last synthetic step. We intended to obtain it by recrystallization but were unable.

It has been found now that rimonabant can exist in a new crystalline form which differs from the other two in their physical properties, in their spectral characteristics and in their methods of preparation.

The new crystalline form (called form III) can be easily obtained by recrystallization in methanol and the process to obtain it can be scaled to industrial scale.

The physical properties can be influenced by controlling the conditions under which Rimonabant is obtained in solid form. Solid state physical properties include, for example, the flowability of the milled solid. Flowability affects the ease with which the material is handled during processing into a pharmaceutical product.

Another important solid state property of a pharmaceutical compound is its rate of dissolution in aqueous fluid. The rate of dissolution of an active ingredient in a patient's stomach fluid can have therapeutic consequences since it imposes an upper limit on the rate at which an orally-administered active ingredient can reach the patient's bloodstream. The solid state form of a compound can also affect its behavior on compaction and its storage stability.

These practical physical characteristics are influenced by the conformation and orientation of molecules in the unit cell, which define a particular polymorphic form of a substance. The polymorphic form can give rise to thermal behavior different from that of the amorphous material or another polymorphic form. Thermal behavior is measured in the laboratory by such techniques as thermogravimetric analysis (TGA), and differential scanning calorimetry (DSC) and can be used to distinguish some polymorphic forms from others. A particular polymorphic form can also lead to distinct spectroscopic properties that can be detectable by powder x-ray crystallography, solid state ¹³C NMR spectrometry, and infrared spectrometry.

In the formulation of drug compositions, it is important for the drug substance to be in a form in which it can be conveniently handled and processed. This is of importance, not only from the point of view of obtaining a commercially viable manufacturing process, but also from the point of subsequent manufacture of pharmaceutical formulations comprising the active compound. Chemical stability, solid state stability, and shelf life of the active ingredients are also very important factors. The drug substance, and compositions containing it, should be capable of being effectively stored over appreciable periods of time, without exhibiting a significant change in the active component's physico-chemical characteristics (e. g. its chemical composition, density, hygroscopicity and solubility).

Moreover, it is also important to be able to provide drug in a form which is as pure as possible. Amorphous materials may present significant problems in this regard. For example, such materials are typically more difficult to handle and to formulate than crystalline material, provide for unreliable solubility, and are often found to be unstable and chemically impure. The skilled person will appreciate that, if a drug can be readily obtained in a stable crystalline form, the above problems may be solved. Thus, in the manufacture of commercially viable and pharmaceutically acceptable, drug compositions, it is desirable, wherever possible, to provide drug in a substantially crystalline, and stable, form. It is to be noted, however, that this goal is not always achievable. Indeed, typically, it is not possible to predict, from molecular structure alone, what the crystallisation behaviour of a compound will be, and this can usually only be determined empirically.

Generally, amorphous form and forms with low crystallinity can exhibit improved solubility, hygroscopicity, bulk properties and/or flowability.

New polymorphic forms of a pharmaceutically compound according to this invention provide a new opportunity to improve the performance characteristics of a pharmaceutical product.

### SUMMARY OF THE INVENTION

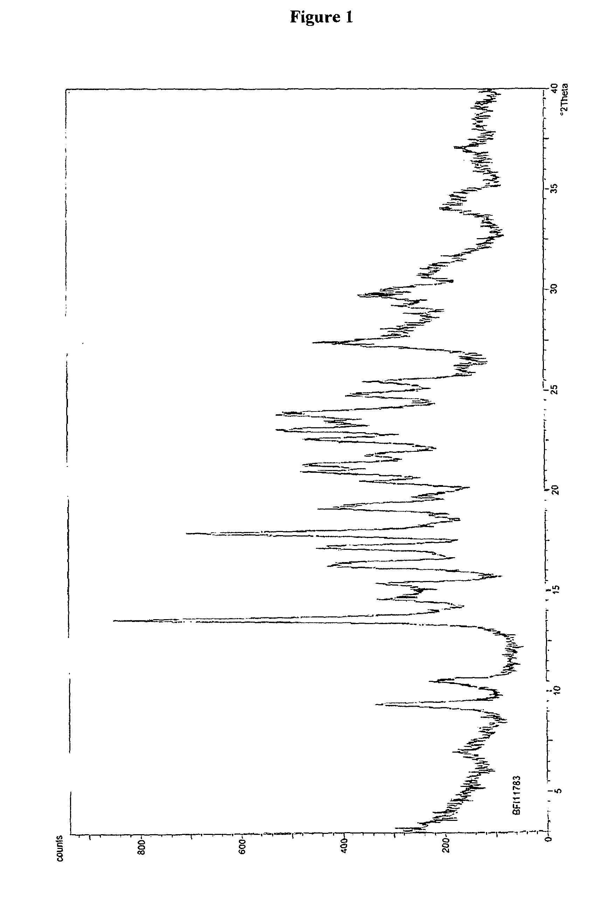

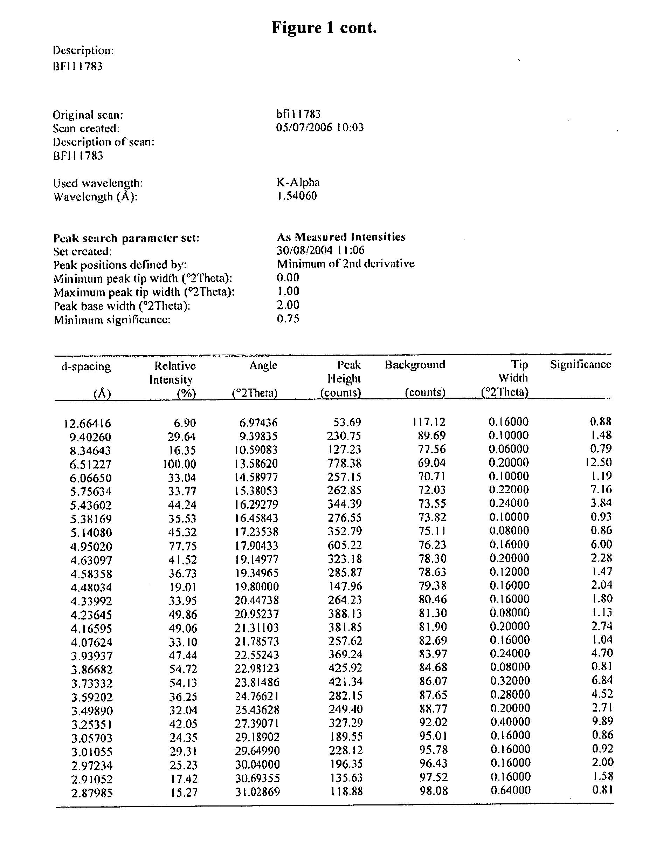

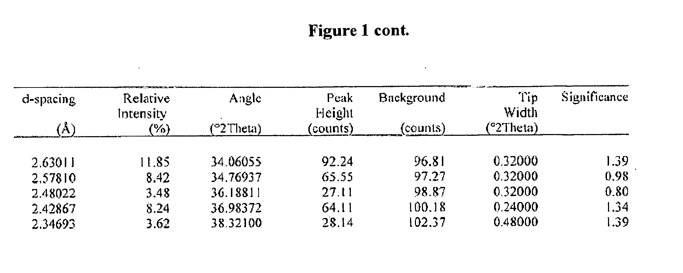

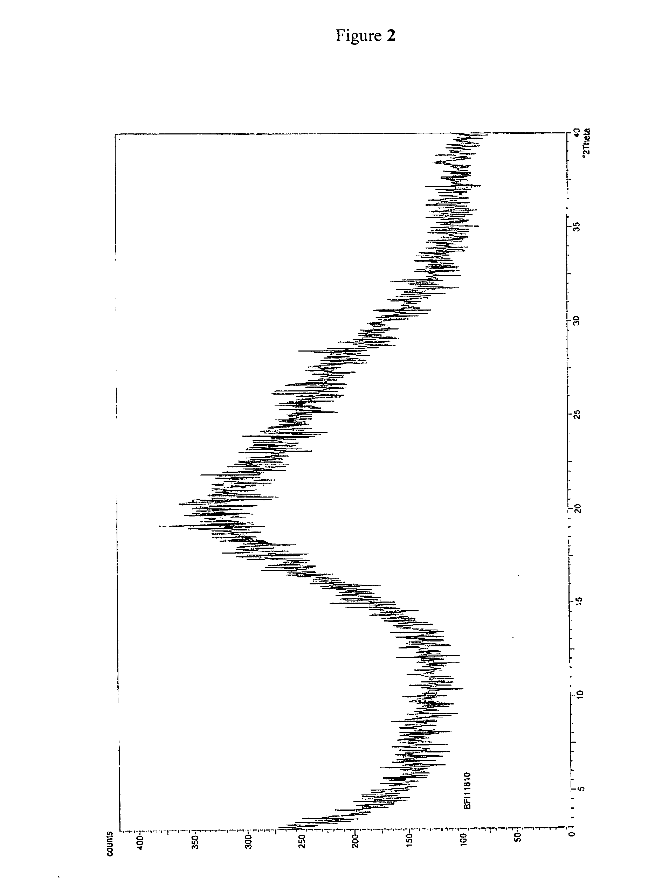

In one aspect, the present invention provides new crystalline forms of Rimonabant (Figure 1), an amorphous form of Rimonabant (Figure 2), and processes for preparing these forms.

In this application, it is disclosed that the form III is isolated according to conventional techniques; more precisely, according to the embodiments exemplified, the product is obtained from the corresponding hydrochloride by acid base reaction with KOH and that the obtained crude compound is crystallizated from methanol. On the other hand, the amorphous form is obtained from methylene dichloride by evaporation solvent.

The method claimed in the present document allows the preparation of rimonabant in crystalline form which will be called form III.

It has now been found that by using particular crystallization conditions, a novel crystalline form called form III is obtained and by using particular evaporation condition an amorphous form is obtained.

In another aspect, the present invention provides solid crystalline Rimonabant denominated Form III characterized by x-ray powder diffraction reflections at 9.4; 10.6; 13.6; 14.6; 15.4; 16.3; 16.5; 17.3; 17.9; 19.2 and 21.0 ± 0.2 degrees two-theta.

The X-ray (XR) powder diffractograms for the crystalline form III and the amorphous form were recorded. The X-ray powder diffraction profile (diffraction angle) was establish with a Philips model X'Pert controlled by a PW3710 unity. Cu Kα radiation and a secondary graphite monochromator were used.

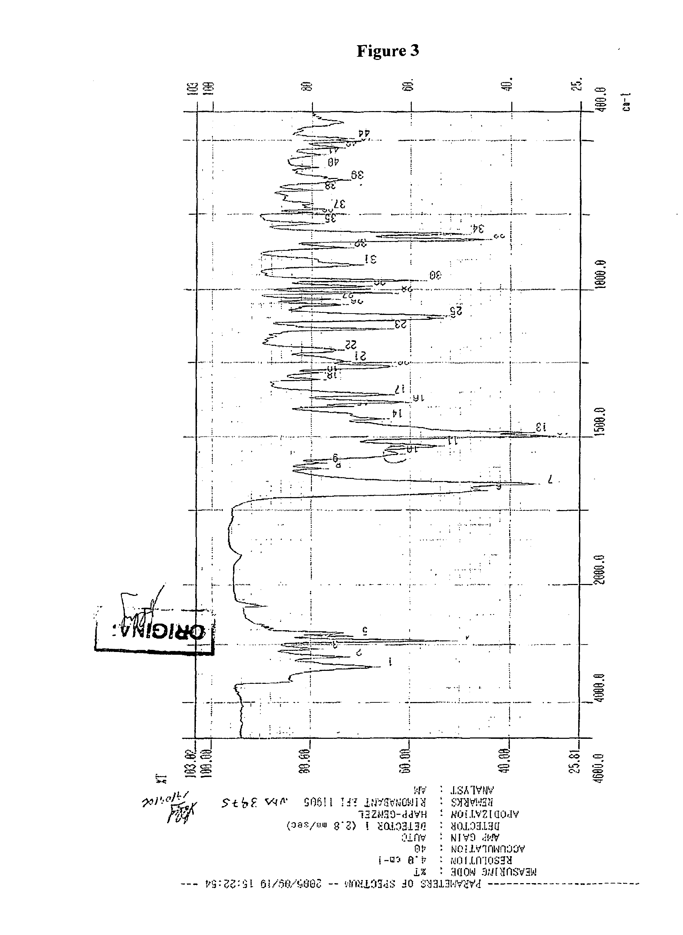

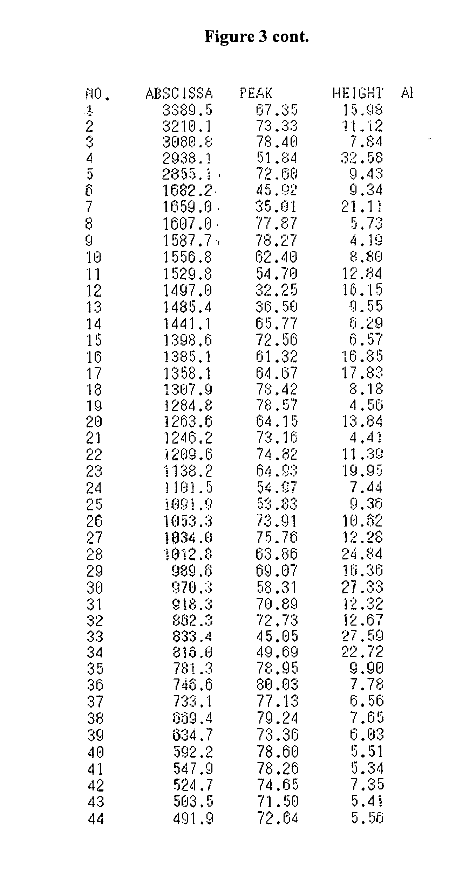

In another aspect. the present invention provides solid crystalline Rimonabant denominated Form III characterized by the absorption bands infrared spectrum at 3389 - 3080, 1682, 1659, 1556, 1529, 1497, 1485, 1101, 1091, 833 and 816 cm⁻¹ among others. (Figure 3)

The infrared spectra were obtained in KBr pellets on a Shimadzu FT-IR 8101M.

In another embodiment, the present invention provides solid amorphous Rimonabant and its representative powder x-ray diffraction diagram is shown in Figure 2.

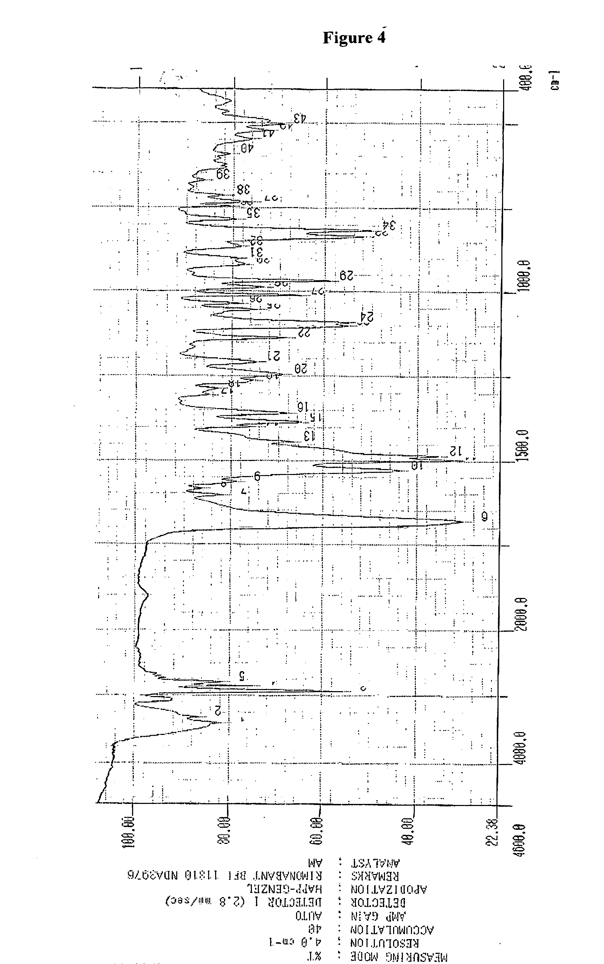

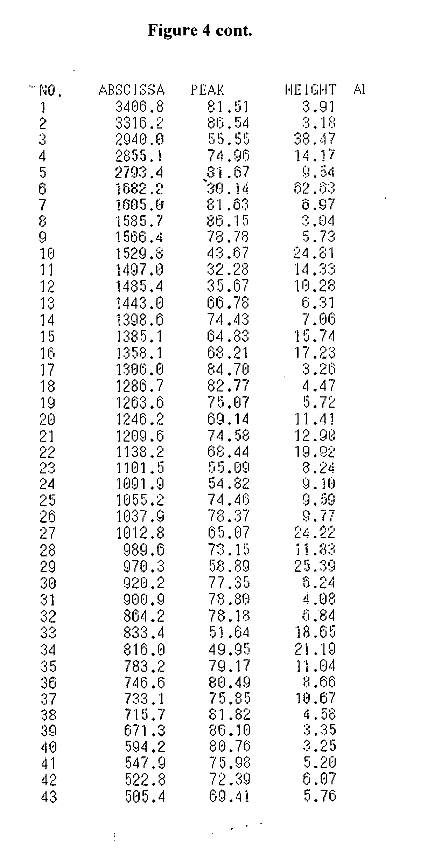

In another aspect, the present invention provides an amorphous form of Rimonabant characterized by the absorption bands infrared spectrum at 1682, 1529, 1497, 1485, 1101, 1091, 833 and 816 cm⁻¹ among others. (Figure 4)

In another embodiment, the present invention provides processes for preparing crystalline forms of Rimonabant including the steps of combining potasium hydroxide with, preferably Rimonabant Hydrochloride, with a solvent and isolating the crystalline form of Rimonabant from the combination. The solvent can be an organic solvent such as C₁-C₃ alcohol or acetonitrile; water; or a mixture thereof. Preferred solvents for use in this embodiment of the present invention include methanol, ethanol, isopropanol, water, and mixtures thereof. The potassium hydroxide can be solid, aqueous, or preferably, the potasium hydroxide is in solution in the solvent with which the potasium hydroxide and Rimonabant are combined. The crystalline Rimonabant is preferably precipitated from a solution.

The processes can also comprise heating the combination (which can be a solution) and/or cooling the combination. For example, the combination can be heated to a temperature above room temperature up to a temperature of about 50°C to about 70°C, preferably about reflux temperature. The combination can be cooled to a temperature of about room temperature to about O°C, preferably about 5°C. The solution can be cooled at once or stepwise. When a cooling step is used with the processes beginning with Rimonabant Hydrochloride and KOH, the solution is preferably cooled stepwise, most preferably by first cooling to room temperature and then cooling further with an ice bath.

Isolating the crystalline form can be performed by any means known in the art. For example, the crystalline form can be isolated by suction filtration. The processes can also include washing and/or drying the precipitated crystalline form. For example, the crystalline form can be washed with the same solvent used for dissolution and/or water. It can be dried to constant weight at 50-70°C.

The crystalline form III of rimonabant is particularly suitable for the manufacture of pharmaceutical composition useful for treating any diseases in which an antagonist of CB₁ cannabinoid receptors is involved.

According to another aspect, the subject of the present invention is pharmaceutical compositions containing, as active ingredient, rimonabant in crystalline form III or in an amorphous form.

In the pharmaceutical compositions of the present invention for administration by the oral, sublingual, subcutaneous, intramuscular, intravenous, transdermal or local route, the active ingredient alone or in combination with another active ingredient, can be administered in single-dose administration forms, as a mixture with conventional pharmaceutical vehicles, to animals and human beings. The appropriate single-dose administration forms comprise the forms by the oral route, such as tablets, gelatin capsules, pills, powders, granules and oral solutions or suspensions, sublingual and buccal administration forms, aerosols, implants, local, transdermal, subcutaneous, intramuscular, intravenous, intranasal or intraocular administration forms.

In the pharmaceutical composition of the present invention, the active ingredient or active ingredients are generally formulated as dosage unit. The dosage unit contains 0.5 to 1000 mg, advantageously from 5 to 50 mg, preferably from 5 to 35 mg per dosage unit, for daily administrations, once or several times per day.

Although these dosages are examples of average situations, there may be specific cases where higher or lower dosages are appropriates; such dosages also form part of the invention.

The tablets can be prepared by various techniques: direct compression, dry granulation, or wet granulation. The tablets can be coated with various polymers or other appropriated materials. The tablets can have a flash, immediate, delayed or sustained release by preparing polymeric matrices or by using specific polymers when forming the thin film coating.

The gelatin capsules may be soft or hard and may be or may be not coated with a thin film, so as to have a flash, immediate, sustained o delayed activity. They can comprise not only a solid formulation but also liquids or semisolids.

The water-dispersible powders or granulates can comprise the active ingredient or active ingredients as a mixture with dispersing agents, wetting agents or suspending agents, such as polyvinylpyrrolidone or polyvidone, as well as with sweeteners or taste corrigents.

The active agent or active agents can also be formulated in the form of microcapsules or microspheres, optionally with one or more vehicles or additives.

When a solid composition is prepared in the form of tablets or capsules, a mixture of pharmaceutical excipients is added to the active ingredients, which mixture can be compose of diluents, such as, for example, lactose, mannitol, microcrystalline cellulose, starch or dicalcium phosphate, of binders such as, for example, polyvinylpyrrolidone or hydroxypropylmethylcellulose, of disintegrating agent, such as crosslinked polyvinylpirrolidone or crosslinked carboxymethylcellulose, croscarmelose sodium, of flow agents, such as silica or talc, or of lubricants, such as magnesium stearate, stearic acid or sodium stearylfumarate. Wetting agents or surfactants, such as sodium lauryl sulfate, polysorbate 80, poloxamer 188, can be added to the formulation.

The following examples illustrate, but do not pretend to limit the different variants of the polymorphic forms and amorphous form which are appropriate to be used in this invention as is defined in this documentation. The examples defined do in no way limit its overall scope.

### EXAMPLE 1

### Solid Phase III

A solution of KOH (10,6 g) in methanol (87 mL) are added to a solution of Rimonabant hydrochloride (86.87 g) in methanol (347 mL). During the addition the internal temperature is maintained at 0-5°C. Once the addition is completed, water (174 mL) is added. The resulting suspension is cooled to 0-5°C and stirred during 1hs to the mentioned temperature.

The solid is filtered and washed with water (3 x 300 mL). The solid is dried under vacuum at 50-55°C until to constant weight. In this trial, 74.50 g of crude solid is obtained (Yield: 93%)

The crude solid is purified by recrystallization in methanol.

Crude Rimonabant. (72.45 g) and methanol (216 mL) are heated to reflux until to dissolution. After that, activated charcoal (5.80 g) are added and the resulting suspension is hot filtered.

The resulting filtrate is cooled to room temperature, and after that is cooled to 0-5°C during 1 h.

The crystals formed are filtrated, washed with methanol (18 mL) and dried until to constant weight at 70°C.

Rimonabant solid phase III (61.3 g) is obtained as an off-white solid (yield: 85%)

### Example II

### Amorphous Phase

Crude solid (3 g) obtained in Example 1, is dissolved in methylene hydrochloride (12 mL). The resulting solution is evaporated to dryness under vacuum at 50-55°C. Amorphous Rimonabant (2.9 g) is obtained. (Yield: 96%).

### EXAMPLE III

### Pharmaceutical composition

95 mg tablets are manufactured, each containing 20 mg of active ingredient:

### Composition:

| | |
|---|---|
| Rimonabant solid phase III | 20.70 mg |
| colloidal silica | 30.00 mg |
| polyvinylpyrrolidone | 3.800 mg |
| sodium lauryl sulfate | 0.950 mg |
| polyvinylpyrrolidone cross-linked | 4.750 mg |
| Lactose anhydrous | 17.165 mg |
| Maize starch | 16.910 mg |
| Magnesium stearate | 1.425 mg |

All the constituents of the tablet core are mixed together. The homogeneous mixture is either directly compressed or granulated and then compressed to form tablet cores.

### EXAMPLE IV

### Pharmaceutical composition

95 mg tablets are manufactured, each containing 20 mg of active ingredient:

### Composition:

| | |
|---|---|
| Rimonabant amorphous form | 20.70 mg |
| colloidal silica | 30.00 mg |
| polyvinylpyrrolidone | 3.800 mg |
| sodium lauryl sulfate | 0.950 mg |
| polyvinylpyrrolidone cross-linked | 4.750 mg |
| Lactose anhydrous | 17.165 mg |
| Maize starch | 16.910 mg |
| Magnesium stearate | 1.425 mg |

All the constituents of the tablet core are mixed together. The homogeneous mixture is either directly compressed or granulated and then compressed to form tablet cores.

The characteristics presented in the description, in the examples and in the claims can constitute, either separately or in any combination, the material for the realization of the invention in its diverse forms.

It is unquestionable that when putting into practice this invention, changes may be introduced as related to certain construction details and form, without this implying getting off the main principles clearly substantiate in the claims following hereinunder.

## Claims

1. Crystalline form III of rimonabant, **characterized by** the following X-ray powder diffractogram lines: 13.6, 14.6, 17.3, and 21.0 ± 0.2 degrees two-theta.

2. Crystalline form III of rimonabant, **characterized by** the following X-ray powder diffractogram lines: 9.4; 10.6; 13.6; 14.6; 15.4; 16.3; 16.5; 17.3; 17.9 ;19.2 and 21.0 ± 0.2 degrees two-theta.

3. Crystalline form III of rimonabant, **characterized in that** its X- rays powder diffractogram is the one corresponding to figure 1.

4. Crystalline form III of rimonabant, **characterized by** the following absorption bands in infrared spectrum are. 3389 - 3080, 1682, 1659, 1556, 1529, 1497, 1485, 1101, 1091, 833 and 816 cm⁻¹ among others.

5. Crystalline form III of rimonabant, **characterized by** the absorption bands infrared spectrum is the one corresponding to figure 3.

6. An amorphous form of rimonabant, **characterized by** the following absorption bands in infrared spectrum are: 1682, 1529, 1497, 1485, 1101, 1091, 833 and 816 cm⁻¹ among others.

7. An amorphous form of rimonabant, **characterized by** the following absorption bands in infrared spectrum is the one corresponding to figure 4.

8. An amorphous form of rimonabant, **characterized in that** its X- rays powder diffractogram is the one corresponding to figure 2.

9. A method for producing the crystalline form III of rimonabant according to any one of claims 1 to 5 which is **characterized in that**
➢ rimonabant hydrochloride is dissolved in methanol, water or a mixture of these solvents;
➢ Potassium hydroxide in methanol is added
➢ where appropriate, the medium is cooled to a temperature of between 0° C and 25° C
➢ the crystals formed are filtered at ambient temperature of between 0° C and 25° C
➢ the solid is vacuum dried at 30-80°C
➢ the solid is crystallized from methanol.

10. A method for producing the amorphous form of rimonabant according to claim 6 to 8 which is **characterized in that**
➢ rimonabant hydrochloride is dissolved in methanol, water or a mixture of these solvents;
➢ Potassium hydroxide in methanol is added
➢ where appropriate, the medium is cooled to a temperature of between 0° C and 25° C
➢ the crystals formed are filtered at ambient temperature of between 0° C and 25° C
➢ the solid is vacuum dried at 30-80°C
➢ the solid is dissolved in methylene dichloride and the result solution is evaporated to obtain the solid.

11. A method according to claim 9, wherein the yield of the crystalline form III of rimonabant is at least 85%.

12. A method according to claim 10, wherein the yield of the amorphous form of rimonabant is at least 96%.

13. Crystalline form of Rimonabant (form III) prepared by the method for producing of claim 9.

14. Crystalline form of Rimonabant (form III) according to any one of claims 1 to 5 having a yield of at least 85%.

15. An amorphous form of Rimonabant prepared by the method for producing of claim 10.

16. An amorphous form of Rimonabant according to any one claims 6 to 8 having a yield of at least 96%.

17. Pharmaceutical composition containing, as active ingredient, the crystalline form of rimonabant (form III) according to claims 1 to 5 in combination with at least one pharmaceutical excipient.

18. Pharmaceutical composition containing, as active ingredient, an amorphous form of rimonabant according to claims 6 to 8 in combination with at least one pharmaceutical excipient.

19. Pharmaceutical composition containing a pharmaceutically effective amount of the crystalline form of rimonabant (form III) according to claims 1 to 5 or one of its pharmaceutically acceptable salts, mixed at least one pharmaceutically acceptable excipient.

20. Pharmaceutical composition containing a pharmaceutically effective amount of an amorphous form of rimonabant according to claims 6 to 8 or one of its pharmaceutically acceptable salts mixed at least one pharmaceutically acceptable excipient.

21. Pharmaceutical composition containing a pharmaceutically effective amount of the crystalline form of rimonabant (form III) according to claims 1 to 5 or one of its pharmaceutically acceptable salts which is in the form of a dosage unit.

22. Pharmaceutical composition containing a pharmaceutically effective amount of an amorphous form of rimonabant according to claims 6 to 8 or one of its pharmaceutically acceptable salts which is in the form of a dosage unit.

23. Pharmaceutical composition containing a pharmaceutically effective amount of the crystalline form of rimonabant (form III) according to claims 1 to 5 or one of its pharmaceutically acceptable salts containing from 0.5 to 1000 mg of active principle.

24. Pharmaceutical composition containing a pharmaceutically effective amount of an amorphous form of rimonabant according to claims 6 to 8 or one of its pharmaceutically acceptable salts containing from 0.5 to 1000 mg of active principle.
